# EUROPÄISCHE PATENTANMELDUNG

(11) **EP 3 739 055 A1**
(43) Veröffentlichungstag der Anmeldung: **18.11.2020**
(21) Anmeldenummer: 19174737.7
(22) Anmeldetag: 15.05.2019
(51) Int. Cl.: C12P 7/04

(54) **BIOTECHNOLOGISCHE HERSTELLUNG VON AROMASTOFFEN AUS JOHANNISBEERTRESTER**

(71) Anmelder: Symrise AG, 37603 Holzminden (DE)
(72) Erfinder: Zorn, Holger, 35435 Wettenberg (DE); Sommer, Svenja, 35392 Gießen (DE); Sella, Nadine, 35415 Pohlheim (DE); Schlering, Christine, 97199 Ochsenfurt (DE); Rühl, Martin, 61209 Echzell (DE); Fraatz, Marco, 35392 Gießen (DE); Büttner, Julia, 35390 Gießen (DE)
(74) Vertreter: Eisenführ Speiser

(57) **Zusammenfassung**

Die vorliegende Erfindung betrifft ein biokatalytisches Verfahren zum Herstellen eines Aromastoffes oder einer Mischung von Aromastoffen auf Basis eines Umsetzungsmediums mit einem auf Pflanzenteilen der Stachelbeergewächse basierenden Bestandteil. Die Erfindung betrifft zudem die Verwendung eines Umsetzungsmediums mit einem auf Pflanzenteilen der Stachelbeergewächse basierenden Bestandteil und/oder eines Biokatalysators zum Herstellen eines Aromastoffes oder einer Mischung von Aromastoffen. Des Weiteren betrifft die vorliegende Erfindung Zusammensetzungen umfassend Verbindungen, die mittels des biokatalytischen Verfahrens herstellbar sind, sowie deren Verwendung in einer der Ernährung, der Kosmetik, der Hygiene oder dem Genuss dienenden Zubereitung.

## Beschreibung

Die vorliegende Erfindung betrifft das Gebiet der Aromastoffe. Insbesondere betrifft die vorliegende Erfindung ein Verfahren zum Herstellen eines Aromastoffes oder einer Mischung von Aromastoffen wie hierin beschrieben. Die Erfindung betrifft zudem die Verwendung eines Umsetzungsmediums mit einem auf Pflanzenteilen der Stachelbeergewächse basierenden Bestandteil wie hierin beschrieben und/oder eines Biokatalysators wie hierin beschrieben zum Herstellen eines Aromastoffes oder einer Mischung von Aromastoffen. Des Weiteren betrifft die vorliegende Erfindung Zusammensetzungen umfassend Verbindungen wie hierin beschrieben und deren Verwendung in einer der Ernährung, der Kosmetik, der Hygiene oder dem Genuss dienenden Zubereitung wie hierin beschrieben.

Natürliche Aromastoffe werden klassisch durch Extraktion aus Pflanzen gewonnen. Als Beispiel sei hier Linalool genannt. Linalool ist ein einwertiger Alkohol, der zur Gruppe der Terpene gehört. Die Verbindung ist farblos, brennbar und hat einen ausgeprägten wohlriechenden Geruch. Bekannte und reiche Vorkommen von Linalool sind Kräuter wie Basilikum, Bohnenkraut, Koriander, Oregano oder Thymian. Linalool kommt für eine Vielzahl von Anwendungen in Betracht. Wegen dessen parfümierender und/oder desodorierender Funktion wird die Verbindung beispielsweise als Geschmacksstoff, in Aromaölen oder in der Parfümindustrie als Ersatz für Bergamot verwendet. Außerdem ist die Verbindung als Aroma in Wein anzutreffen.

Um den starken und stetig wachsenden Bedarf an Linalool decken zu können, wird heutzutage ein nicht unbedeutender Anteil des Marktvorkommens chemisch synthetisiert. Zwar bergen chemische Synthesen die Möglichkeit der Herstellung großer Mengen an Aromastoffen, allerdings sind viele Synthesen wenig umweltverträglich. Insbesondere die meist zu vernachlässigenden Reaktionsraten unter Normalbedingungen führt dazu, dass akzeptable Ausbeuten nur durch Anwendung harscher Synthesebedingungen wie hohe Temperaturen und hohe Drücke erzielt werden können. Des Weiteren setzen chemische Synthesen nicht selten Schwermetallkatalysatoren, brennbare Gase und organische Lösemittel ein, deren Verwendung mit bekannten Nachteilen behaftet ist. Aus diesem Grund besteht ein wachsender Bedarf an alternativen Herstellungsverfahren für Linalool, aber auch für andere aromawirksame Verbindungen, die die Nachteile der bekannten Verfahren wenigstens zum Teil überwinden.

Abhilfe könnten biotechnologische Verfahren schaffen, die auf einer Biotransformation natürlicher Vorläufersubstanzen zu gewünschten Aromastoffen mit Mikroorganismen oder deren Enzymen basieren (Berger, R. G., Flavors and Fragrances: Chemistry, Bioprocessing and Sustainability, (2007), Berlin Heidelberg, Springer Verlag). Großes Potential für die biotechnologische Herstellung von Linalool und anderen Aromastoffen könnten dabei insbesondere landwirtschaftliche Nebenströme haben, die zum Teil reich an Ballaststoffen und Sekundärmetaboliten als potentielle Aromapräkursoren sind.

Es war deshalb die primäre Aufgabe der vorliegenden Erfindung, den oben beschriebenen Nachteilen abzuhelfen und ein Verfahren zum Herstellen von Aromastoffen wie Linalool bereitzustellen. Insbesondere war es ein Anliegen, ein Verfahren bereitzustellen, das umweltverträglicher als die bekannten chemischen Synthesen ist.

Erfindungsgemäß gelöst wird diese primäre Aufgabe durch ein Verfahren zum Herstellen eines Aromastoffes oder einer Mischung von Aromastoffen, umfassend die Schritte: Bereitstellen eines Umsetzungsmediums (hier teilweise auch als Kulturmedium oder Substrat bezeichnet) mit einem auf Pflanzenteilen der Stachelbeergewächse (Grossulariaceae) basierenden Bestandteil;
Inkontaktbringen des Umsetzungsmediums mit einem Biokatalysator;
Umsetzen des zumindest auf Pflanzenteilen der Stachelbeergewächse basierenden Bestandteils zu dem Aromastoff oder der Mischung von Aromastoffen mithilfe des Biokatalysators; und gegebenenfalls
Gewinnen des Aromastoffs oder der Mischung von Aromastoffen.

Weitere Aufgaben, Aspekte und bevorzugte Ausgestaltungen der vorliegenden Erfindung ergeben sich aus den nachfolgenden Ausführungen, den beigefügten Beispielen, den Figuren und insbesondere den beigefügten Patentansprüchen.

Die Figuren zeigen:

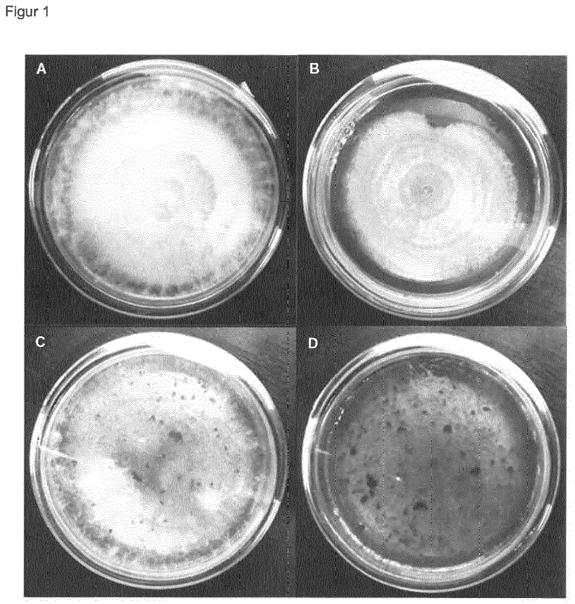

Figur 1: Vergleich einer Umsetzung auf erfindungsgemäßem Kulturmedium (Umsetzungsmedium) gegenüber Standardkulturmedium am Beispiel des Pilzes *W*. *cocos.* Dargestellt sind Fotografien von Emerskulturen von *W. cocos* nach 11 Tagen (d) auf unterschiedlichen Kulturmedien. A: Malzextrakt-Agar (MEA), B: MEA + 0,624% Mononatrium-L-Aspartat, C: 3% Trester, D: 3% Trester + 0,624% Mononatrium-L-Aspartat.

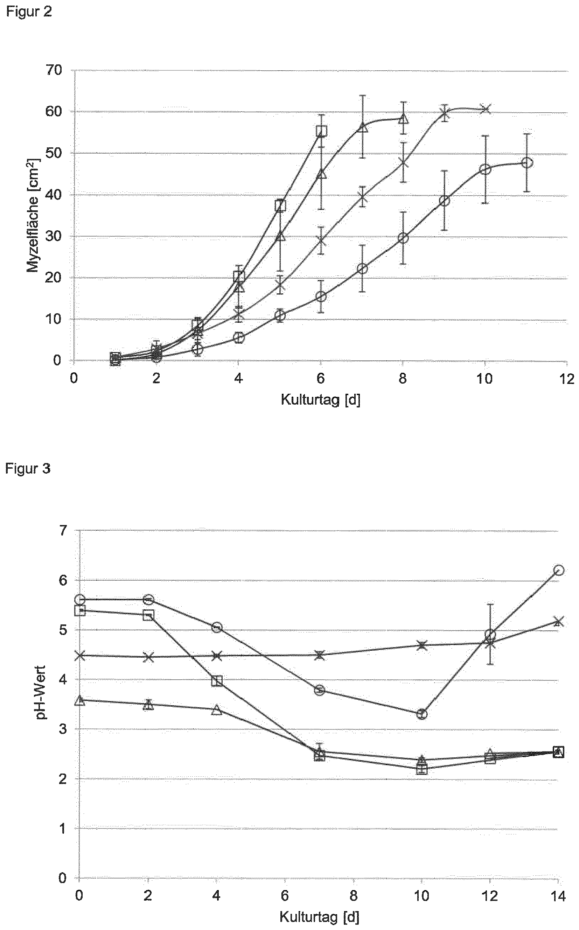

Figur 2: Vergleich einer Umsetzung auf erfindungsgemäßem Kulturmedium gegenüber Standardkulturmedium am Beispiel des Pilzes *W*. *cocos.* Dargestellt sind Wachstumskurven der Emerskulturen von *W. cocos* auf vier verschiedenen Medien: MEA (□); MEA + Asp (Δ); 3TT (x); 3TT + Asp (○). Verwendete Abkürzungen: 3TT : 3% Trester der Sorte Titania, Asp: 0,624% Mononatrium-L-Aspartat.
Figur 3: Vergleich einer Umsetzung auf erfindungsgemäßem Kulturmedium gegenüber Standardkulturmedium am Beispiel des Pilzes *W. cocos.* Dargestellt ist der pH-Wert-Verlauf der Medien während der Emerskultivierung von *W*. *cocos* auf MEA (□); MEA + Asp (Δ); 3TT (x); 3TT + Asp (○). Verwendete Abkürzungen wie zuvor beschrieben.

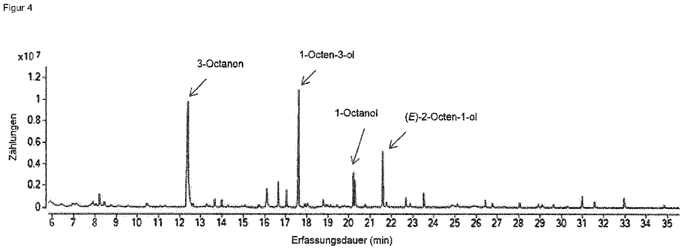

Figur 4: GC-MS-Chromatogramm (Messung splitless) einer Umsetzung auf nicht-erfindungsgemäßem Kulturmedium am Beispiel einer Fermentation von *W*. *cocos* auf MEA. Dargestellt ist das Chromatogramm einer nach 8 Kulturtagen gezogenen Probe mit Substanzvorschlägen.

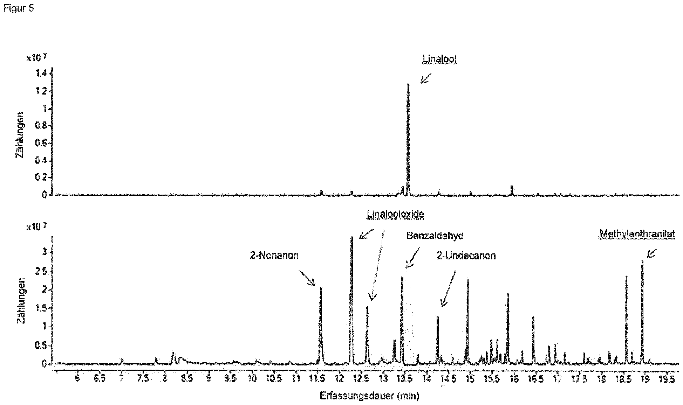

Figur 5: GC-MS-Chromatogramme einer Umsetzung auf erfindungsgemäßem Kulturmedium am Beispiel einer Emersfermentation von *W. cocos* auf 3% Johannisbeertrester, 0,624% Mononatrium-L-Aspartat, 3% Agar-Agar. Dargestellt ist das Chromatogramm einer nach 11 Kulturtagen gezogenen Probe; oben: Messung mit einem Split von 50:1, unten splitless, wobei der MS-Detektor zwischen 13,5 min - 13,7 min ausgeschaltet wurde (grau hinterlegt).

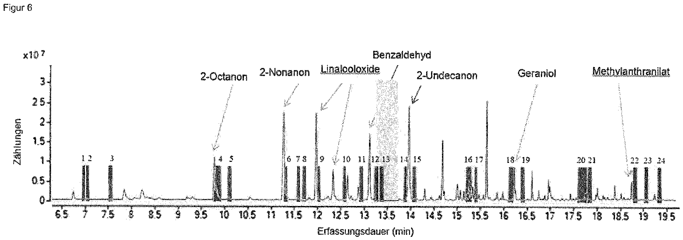

Figur 6: GC-Chromatogramm mit ODP-Spur und Substanzvorschlägen einer Umsetzung auf erfindungsgemäßem Kulturmedium am Beispiel einer Emersfermentation von *W*. *cocos* auf 3% Johannisbeertrester und 0,624% Mononatrium-L-Aspartat. Dargestellt ist das Chromatogramm einer nach 10 Kulturtagen gezogenen Probe; zwischen 13,2 min und 13,7 min war der MS-Detektor deaktiviert (grau hinterlegt). Schwarze mit Zahlen versehene Banden geben typischerweise zu erwartende Gerüche gemäß der nachfolgenden Übersicht an.

| | | | |
|---|---|---|---|
| 1 | grün | 13 | blumig, Citrus, frisch |
| 2 | Käse | 14 | grün |
| 3 | Lösungsmittel | 15 | süßlich, blumig |
| 4 | süßlich, grün | 16 | grün, frisch, Citrus |
| 5 | pilzig, Champignon | 17 | grün, Tanne |
| 6 | grün, süßlich, blumig | 18 | Tanne, blumig |
| 7 | pilzig | 19 | Lösungsmittel, süßlich |
| 8 | grün, blumig | 20 | dumpf, unangenehm, gebraten |
| 9 | dumpf, grün, Tanne, blumig | 21 | Karamell |
| 10 | dumpf | 22 | fruchtig |
| 11 | dumpf, grün, blumig | 23 | dumpf, grün |
| 12 | Marzipan, süßlich | 24 | grün |

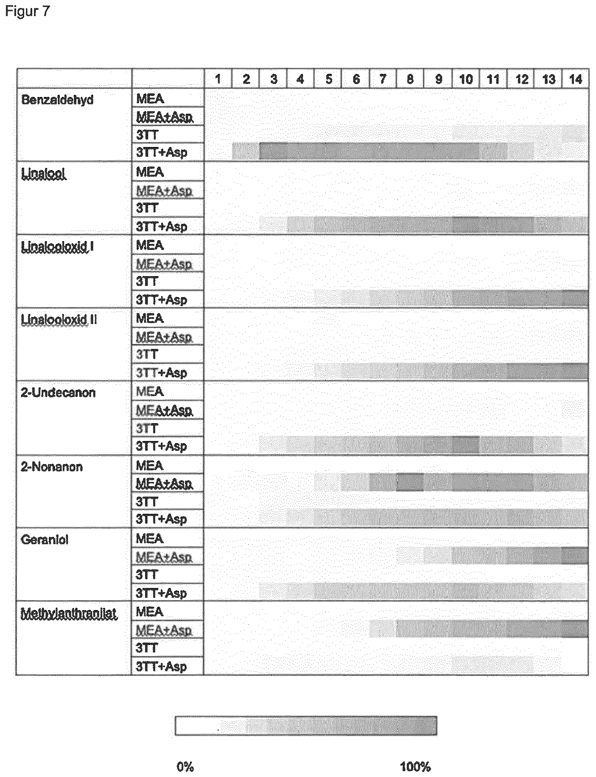

Figur 7: Heatmap ausgewählter Aromen bei der Kultivierung von *W*. *cocos* auf verschiedenen Medien; relative Veränderung während der 14 Kulturtage. Als Grundlage dienten die Peakflächen der EIC bei m/z(Benzaldehyd) = 106, m/z(Linalool) = 93, m/z(Linalooloxid I, II) = 111, m/z(2-Undecanon) = 71, m/z(2-Nonanon) = 58, m/z(Geraniol) = 69, m/z(Methylanthranilat) = 119. Die Zuordnung der jeweiligen Sättigungsstufe eines ausgewählten Fragments erfolgte gemäß unten dargestelltem Schema auf Basis der relativen Peakfläche der EIC (0% bis 100%) des ausgewählten Fragments zum jeweiligen Zeitpunkt (1 bis 14 Tage).

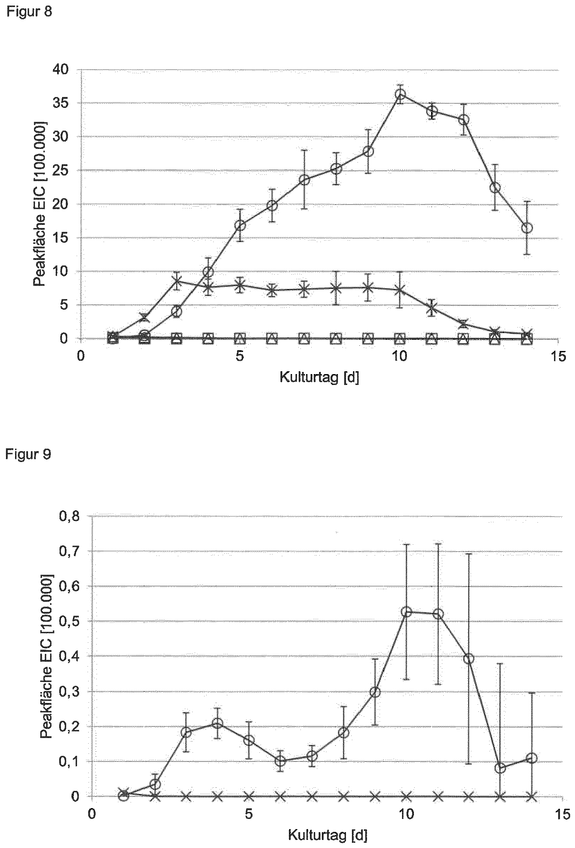

Figur 8: Entwicklung des Aromaprofils von Linalool und Benzaldehyd während der Umsetzung auf erfindungsgemäßem Kulturmedium im Vergleich zu einer entsprechenden Umsetzung auf Standardkulturmedium am Beispiel einer Fermentation von *W. cocos.* Entwicklung von Linalool auf MEA (□), Entwicklung von Linalool auf 3TT+Asp (○); Entwicklung von Benzaldehyd auf MEA (Δ); Entwicklung von Benzaldehyd auf 3TT+Asp (x). Für die Erstellung des EIC wurde für Linalool das Fragment bei m/z = 93, für Benzaldehyd m/z = 106 gewählt. Verwendete Abkürzungen wie zuvor beschrieben.
Figur 9: Entwicklung des Aromaprofils von Methylanthranilat (m/z = 119) während der Umsetzung auf erfindungsgemäßem Kulturmedium im Vergleich zu einer entsprechenden Umsetzung auf Standardkulturmedium am Beispiel einer Fermentation von *W. cocos.* Entwicklung von Methylanthranilat auf MEA (x); Entwicklung von Methylanthranilat auf 3TT+Asp (○).

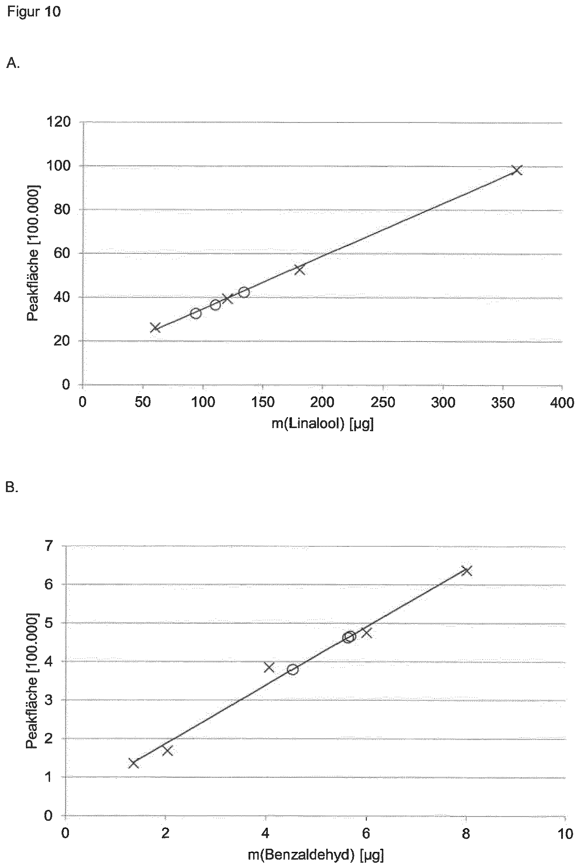

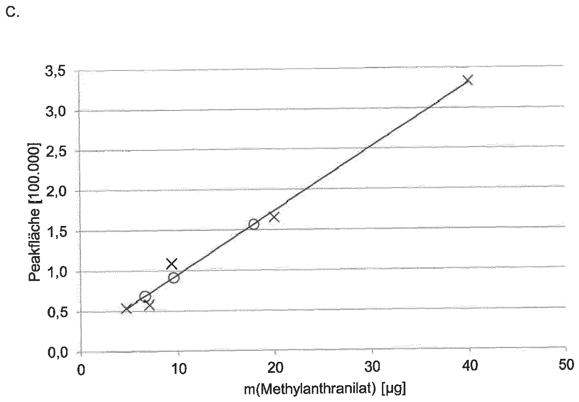

Figur 10: Semiquantitative Abschätzung der Konzentrationen ausgewählter Aromastoffe (○) nach Umsetzung auf erfindungsgemäßem Kulturmedium am Beispiel einer Fermentation von *W*. *cocos* auf 3% Trester mit 0,624% Mononatrium-L-Aspartat. Hierbei wurde der Kopfraum der Kulturen nach 10 d analysiert und mit einer mittels Standards ermittelten Kalibrierreihe (x) verglichen. A. Linalool; B: Benzaldehyd; C: Methylanthranilat. Die abgeschätzte Menge ist in µg pro Platte aufgetragen.

Die Erfindung basiert maßgeblich auf der Erkenntnis, dass ein mit einem auf Pflanzenteilen der Stachelbeergewächse basierenden Bestandteil versehenes Umsetzungsmedium äußerst interessante Aromavorläufersubstanzen enthält, die biokatalytisch zu Aromastoffen umgewandelt werden können. Der Begriff Aromastoff bezeichnet hier eine Verbindung, die in aromawirksamer Menge einen wahrnehmbaren Geschmack oder Geruch vermittelt. Dabei ist mit dem Begriff "aromawirksam" diejenige Menge der Verbindung gemeint, die ausreichend ist, um bei Gebrauch einer die Verbindung enthaltenden Zubereitung einen sensorischen Effekt an Geruchs- und/oder Geschmacksrezeptoren hervorzurufen. Ein derartiger Effekt kann sich auch dadurch äußern, dass eine unangenehme geschmacks- und/oder geruchsbasierte Sinneswahrnehmung vermindert oder maskiert wird.

Das erfindungsgemäße Verfahren vermeidet die Notwendigkeit harscher Herstellungsbedingungen, organischer Lösemittel oder Schwermetallkatalysatoren, die im Falle chemischer Synthesen kaum vermeidbar sind. Die vorliegende Erfindung stellt somit eine umweltfreundliche Alternative zu chemischen Synthesen dar, die darüber hinaus dem wachsenden Bedarf an Aromastoffen wie Linalool gerecht wird.

Ferner zeichnet sich das erfindungsgemäße Verfahren dadurch aus, dass es das Potential bietet, landwirtschaftliche Abfallströme wie Blätter oder Trester der Stachelbeergewächse als Quelle für hochwertige Aromastoffe einzusetzen und so in die Wertschöpfungskette landwirtschaftlicher Produktionen gewinnbringend integriert werden zu können. Aus diesem Grund ist insbesondere ein Verfahren bevorzugt, bei dem der auf Pflanzenteilen der Stachelbeergewächse basierende Bestandteil in Form von Blättern und/oder eines Tresters der Stachelbeergewächse wie hierin beschrieben, und/oder eines Extrakts davon, bereitgestellt wird. Ganz besonders bevorzugt ist die Verwendung eines Tresters der Stachelbeere und insbesondere eines Tresters der schwarzen Johannisbeere wie hierin beschrieben. Der Anteil des auf Pflanzenteilen der Stachelbeergewächse basierenden Bestandteils in erfindungsgemäßen Verfahren wie hierin beschrieben beträgt vorzugsweise 0,2 Gewichts-% bis 20 Gewichts-%, bevorzugt 0,5 Gewichts-% bis 10 Gewichts-%, weiter bevorzugt 1 Gewichts-% bis 5 Gewichts-% und am meisten bevorzugt 2 Gewichts-% bis 4 Gewichts-% bezogen auf das Gesamtgewicht des Umsetzungsmediums.

Der Begriff "Stachelbeergewächse" bezeichnet Gewächse aus der Familie Grossulariaceae. Erfindungsgemäß sind Verfahren bevorzugt, die Pflanzenteile von für die Beerenobstproduktion geeignete Arten und Sorten, insbesondere Arten der Johannisbeeren, Stachelbeeren und deren Kreuzungen wie beispielsweise Josta, verwenden. Weiter bevorzugt sind Verfahren, die Pflanzenteile von Arten der weißen oder roten Stachelbeere, der roten, weißen, schwarzen oder tiefschwarzen Johannisbeeren sowie deren Kreuzungen und Sorten einsetzen. Am meisten bevorzugt verwenden erfindungsgemäße Verfahren Pflanzenteile der schwarzen Johannisbeeren (*Ribes nigrum*) und deren Varietäten und Sorten.

Pflanzenbestandteile umfassen insbesondere oberirdische vegetative oder reproduktive Gewebe, bevorzugt Blätter, Knospen, Blatt- und/oder Blüten-Knospen, Beerenfrüchte und deren Unterbestandteile wie Kern, Schale und Fruchtfleisch ausgewählt sind. Vorzugsweise handelt es sich bei den Pflanzenteilen um Blätter und/oder Beerenfrüchte und weiter bevorzugt um reife Beerenfrüchte als Ganzes.

Ein auf Pflanzenteilen basierender Bestandteil bezeichnet vorliegend einen Bestandteil, der aus Teilen der in Rede stehenden Pflanze gewonnen wurde. Dabei muss der in erfindungsgemäßen Verfahren verwendete Bestandteil nicht notwendigerweise in identischer Weise in der Natur vorkommen. Vielmehr kann ein Bestandteil der jeweiligen Pflanze auch durch Weiterverarbeitung natürlich vorkommender Bestandteile gewonnen werden. Bevorzugte Maßnahmen der Weiterverarbeitung umfassen (teilweises) Trocknen, (teilweises) Gären und/oder (teilweises) Auspressen. Bevorzugt handelt es sich um ein Abfallprodukt aus anderen Industriezweigen, die die entsprechenden Pflanzen oder wenigstens Teile davon als Rohstoff einsetzen. Besonders bevorzugt handelt es sich bei den Pflanzenteilen um Beerenfrüchte (als Ganzes) oder um Rückstände aus der Saftgewinnung (sog. Trester).

Für die vorliegende Erfindung sind insbesondere Geschmacks- und/oder Geruchseindrücke von Interesse, die als angenehm empfunden werden. Die Beurteilung, ob ein Geschmacks- und/oder Geruchseindruck als angenehm oder eher unangenehm gilt, kann durch sensorische Analyse durch ein trainiertes Panel auf Basis einer Bewertung des Sinneseindrucks zwischen negativ (angenehm) und positiv (unangenehm) erfolgen. Zur genaueren Einteilung können weitere Stufen wie sehr negativ, neutral und sehr positiv vorgesehen werden. Die Bestimmung der Noten eines zu beurteilenden Aromastoffs, der in einer Mischung von weiteren Verbindungen, möglicherweise weiteren Aromastoffen vorliegt, kann beispielsweise mittels Gaschromatographie-Olfaktometrie erfolgen. Vorliegend handelt es sich bei dem Aromastoff oder den Aromastoffen insbesondere um solche, die einen angenehmen Geruchseindruck vermitteln und deshalb auch als Riechstoffe bezeichnet werden können.

Im Zusammenhang der Herstellung von Aromastoffen mit angenehmen Geschmacks- und/oder Geruchseindrücken sticht speziell ein Umsetzungsmedium hervor, das einen auf Pflanzenteilen der Johannisbeere, bevorzugt auf Pflanzenteilen der schwarzen Johannisbeere (*Ribes nigrum*) und insbesondere auf Pflanzenteilen der schwarzen Johannisbeere der Sorte Titania basierenden Bestandteil enthält. Dieser Bestandteil enthält augenscheinlich Aromavorläufersubstanzen, aus denen durch biokatalytische Umsetzung als äußerst angenehm empfundene Aromastoffe hergestellt werden können. Das in dem erfindungsgemäßen Verfahren bereitgestellte Umsetzungsmedium enthält deshalb vorzugsweise einen auf Pflanzenteilen der Johannisbeere basierenden Bestandteil, wobei es sich bei der Johannisbeere bevorzugt um die schwarze Johannisbeere und weiter bevorzugt um die schwarze Johannisbeere der Sorte Titania handelt. Die dadurch mittels geeigneter Biokatalysatoren herstellbaren Aromastoffe weisen blumige, frische, fruchtige oder an Waldbeeren oder Citrus erinnernde Noten auf. Diese Aromen sind bei einer entsprechenden Umsetzung auf einem Standardkulturmedium, das auf Malzextrakt basiert, nicht wahrnehmbar. Im Gegenteil: Der olfaktorische Eindruck wurde hier als pilzig, säuerlich und nach tropischen Früchten riechend beschrieben. Weitere Vorteile ergeben sich durch nachfolgend beschriebene Maßnahmen.

Der Anteil des auf Pflanzenteilen der Stachelbeergewächse basierenden Bestandteils in erfindungsgemäßen Verfahren wie hierin beschrieben beträgt vorzugsweise 0,2 Gewichts-% bis 20 Gewichts-%, bevorzugt 0,5 Gewichts-% bis 10 Gewichts-%, weiter bevorzugt 1 Gewichts-% bis 5 Gewichts-% und am meisten bevorzugt 2 Gewichts-% bis 4 Gewichts-% bezogen auf das Gesamtgewicht des Umsetzungsmediums.

Der Schritt des Gewinnens des Aromastoffs oder der Mischung von Aromastoffen umfasst vorzugsweise ein zumindest teilweises Trennen des hergestellten Aromastoffs oder zumindest eines Aromastoffs der Mischung von Aromastoffen von dem Biokatalysator. Bevorzugt erfolgt ein im Wesentlichen vollständiges Abtrennen vom Biokatalysator. Darüber hinaus kann der Schritt des Gewinnens ein Anreichern, Konzentrieren und/oder Isolieren des hergestellten Aromastoffs oder zumindest eines Aromastoffs der Mischung von Aromastoffen umfassen. Der Begriff "zumindest ein Aromastoff" bezeichnet hier insbesondere wahlweise 1, 2, 3, 4, 5, 6, 7 oder 8 Aromastoffe.

Für die Zwecke der vorliegenden Erfindung haben sich besonders ganzzellbasierte Biokatalysatoren aufgrund deren Eigenschaft, sich simultan oder sequentiell zur katalysierten Umsetzungsreaktion zu vermehren, bewährt. So kann der Schritt des Umsetzens auch ein Kultivieren im Sinne eines "Wachsenlassens" oder Vervielfältigens des Biokatalysators umfassen. Dem Fachmann ist jedoch weitläufig bekannt, dass das Umsetzen mittels ganzen Zellen letztendlich auf einer oder mehrerer katalysierten Reaktionen beruht, so dass die gegenwärtige Erfindung auch Verfahren umfasst, bei denen als Biokatalysator zur Herstellung des Aromastoffs oder der Mischung von Aromastoffen auch lediglich die katalytisch relevante Einheit des ganzzellbasierten Biokatalysators zur Herstellung des Aromastoffs oder der Mischung von Aromastoffen enthalten.

Bei dem Biokatalysator handelt es sich vorzugsweise um einen Pilz, eine Pilzzelle davon oder wenigstens eine katalytisch relevante Einheit davon. Eine katalytisch relevante Einheit bezeichnet hier die für das Umsetzen erforderlichen Makromoleküle wie Enzmye oder Ribozyme und ggfs. Cofaktoren und Cosubstrate. Bevorzugt handelt es sich um einen Pilz (oder eine entsprechende Pilzzelle davon), der imstande ist, auf Lignocellulose zu wachsen, und/oder aus der Gruppe der Speisepilze ausgewählt ist/sind. Die Fähigkeit des Wachstums auf Lignocellulose ist deshalb von Vorteil, da sich derartige Pilze besonders gut für ein Kultivieren auf einem auf Stachelbeergewächsen basierenden Bestandteil wie hierin beschrieben eignen. Zudem könnten Aromastoffe wie Benzaldehyd direkt aus dem Abbau von Lignocellulose resultieren. Ein Speisepilz ist im Hinblick auf die Sicherheit bei der Verwendung des hergestellten Aromastoffes oder der hergestellten Mischung von Aromastoffen vorteilhaft. Dieses bevorzugte Anforderungsprofil trifft auf viele Pilze aus der Abteilung der Ständerpilze (*Basidiomycota*) zu. Zudem können mit Pilzen aus der Abteilung der Ständerpilze besonders gute Geruchseindrücke erzielt werden, insbesondere wenn es sich bei der Stachelbeere um eine schwarze Johannisbeere wie hierin beschrieben handelt.

Besonders bemerkenswert ist die Erkenntnis, dass das Umsetzen mit zu den Ständerpilzen gehörenden Spezies zu einem Geruch führt, der im Gesamteindruck als angenehm wahrgenommen wird. Das heißt, pilzige, säuerliche und nach tropischen Früchten riechende Noten, die als typische Pilzaromen bekannt sind, werden nicht oder kaum gebildet. Dementsprechend sind Verfahren bevorzugt, in denen der Pilz aus der Abteilung der Ständerpilze ausgewählt ist und/oder die Pflanzenteile der Stachelbeergewächse aus der Gruppe der Pflanzenteile der Johannisbeeren, bevorzugt solche der schwarzen Johannisbeeren, weiter bevorzugt solche der schwarzen Johannisbeeren der Sorte Titania ausgewählt ist.

Bevorzugt sind insbesondere Verfahren wie hierin beschrieben, wobei der Pilz der Abteilung der Ständerpilze aus der Gruppe bestehend aus *A. campestris, A. aegerita, A. melea, B. adusta, C. comatus, C. limbatus, F. velutipes, G. odoratum, H. fasciculare, I. consors, L. sulphureus, L. edodes, L. nuda, L. pyriforme, M. cohortalis, M. pseudocorticola, M. scorodonius, P. serotinus, P. chrysosporium, P. flabellatus, P. sapidus, S. crispa, S. hirsutum, T. suaveolens, T. chioneus und W. cocos* ausgewählt ist. Diese Spezies sind hinsichtlich Ihrer Fähigkeit, Aromastoffe mit angenehmen Geruchseindrücken zu bilden, einer Vielzahl von weiteren getesteten und hier nicht näher spezifizierten Pilzen bzw. Pilz-Umsetzungsmedium-Kombinationen deutlich überlegen.

Weiter bevorzugt sind Verfahren wie hierin beschrieben, wobei der Pilz ausgewählt ist aus der Gruppe bestehend aus *A. campestris, A. aegerita, C. comatus, G. odoratum, H. fasciculare, I. consors, L. sulphureus, L. edodes, L. pyriforme, M. cohortalis, M. pseudocorticola, M. scorodonius, P. serotinus, P. chrysosporium, P. flabellatus, P. sapidus, S. crispa, T. suaveolens, T. chioneus* und *W. cocos.* Diese Auswahl führt in wenigstens einer möglichen Kultivierungsform zu einem besonders angenehmen Aroma.

Weiter bevorzugt sind Verfahren wie hierin beschrieben, wobei der Pilz ausgewählt ist aus der Gruppe bestehend aus *A. aegerita, C. comatus, G. odoratum, H. fasciculare, L. sulphureus, M. pseudocorticola, M. scorodonius, P. flabellatus, P. sapidus, S. crispa, T. suaveolens* und *W. cocos.* Die vorgenannte Auswahl führt in wenigstens zwei möglichen Kultivierungsformen zu einem besonders angenehmen Aroma.

Weiter bevorzugt sind Verfahren wie hierin beschrieben, wobei der Pilz ausgewählt ist aus der Gruppe bestehend aus *W. cocos* und *G. odoratum.* Die Pilze *G. odoratum* und *W. cocos* zeigen auf allen getesteten Substraten in allen getesteten Kultivierungsformen sehr intensive Geruchseindrücke. *G. odoratum* produziert hierbei ein citrusartiges Aroma, das Aroma von *W*. *cocos* ist blumig, fruchtig und erinnert an Walderdbeeren. Diese Auswahl führt also unabhängig von der möglichen Kultivierungsform zu einem besonders angenehmen Aroma.

Wie vorstehend beschrieben gehören zu den bevorzugten Biokatalysatoren auch einzelne Zellen oder katalytisch wirksame Einheiten der oben genannten bevorzugten und weiter bevorzugten Pilze.

Des Weiteren sind erfindungsgemäß Verfahren bevorzugt, in denen das bereitgestellte Umsetzungsmedium eine Aspartatquelle, bevorzugt eine L-Aspartatquelle, weiter bevorzugt ein Natrium-L-Aspartat Salz und am meisten bevorzugt Mononatrium-L-Aspartat umfasst. So stellte sich im Rahmen der durchgeführten Versuche heraus, dass die Zugabe von Mononatrium-L-Aspartat einem sonst beobachteten pH-Wert Abfall entgegenwirkt und letztendlich zu einem besonders intensiven Geruch nach Walderdbeeren führt. Nach ca. 10 Kulturtagen intensiviert sich der Geruch mit blumigen Noten und einem intensiven fruchtigen Aroma, das an Walderdbeeren erinnert. Die Signalintensitäten der Aromen sind bei Kultivierung auf dem mit Aspartat-supplementierten Umsetzungsmedium wie hierin beschrieben deutlich höher als bei der Kultivierung auf Standardkulturmedium, das auf Malzextrakt basiert. Besonders hoch sind die Intensitäten von Linalool, was gut mit dem blumigen Geruch der Kultur korreliert. Einige aromawirksame Stoffe, wie Benzaldehyd, Linalool, Linalooloxid I, II und 2-Undecanon werden besonders gut auf dem Aspartat-haltigen Umsetzungsmedium, das vorzugsweise in Form eines Tresters wie hierin beschrieben bereitgestellt wird, gebildet. Der Gehalt der Aspartatquelle beträgt vorzugsweise 0,1 % bis 5,0 %, bevorzugt 0,2 % bis 2,0 %, weiter bevorzugt 0,3 % bis 1,0 % und insbesondere 0,5 % bis 0,7 %, jeweils bezogen auf das Gesamtgewicht des Umsetzungsmediums.

Dem pH-Abfall kann alternativ auch mit anderen puffernden Substanzen entgegengewirkt werden. Vorzugsweise liegt der pH-Wert des Umsetzungsmediums während dem Umsetzen innerhalb eines Bereichs von pH 2 bis pH 7, vorzugsweise pH 4 bis pH 6 und insbesondere pH 4,2 bis pH 5,5 liegt. Bei einer über mehrere Tage andauernden Umsetzung bedeutet dies, dass der pH-Wertbereich zumindest im Wesentlichen über den gesamten Zeitraum eingehalten wird.

Zur Optimierung des Aromaprofils und der Intensität können dem Umsetzungsmedium weitere Nährstoffquellen zugefügt werden. Hierzu zählen neben der Aspartatquelle wie hierin beschrieben insbesondere Glucose, Spurenelemente, die aus der Gruppe von Mg²⁺, Zn²⁺, Fe³⁺, Mn²⁺, Cu²⁺, K⁺, Cl⁻, SO₄²⁻ und PO₄³⁻ ausgewählt sind, sowie andere Aminosäuren.

Im Rahmen der vorliegenden Erfindung kommen grundsätzlich alle Kultivierungsformen in Betracht, auch wenn die konkrete Form einen Einfluss auf die gebildeten Aromastoffe haben kann. So werden besonders gute Ergebnisse bei Umsetzen in Emerskultur erzielt, insbesondere wenn als Biokatalysator *W*. *cocos* und/oder Trester als dem auf Stachelbeergewächsen basierenden Bestandteil wie hierin beschrieben eingesetzt wird/werden. In Abhängigkeit der verwendeten Pilzspezies wie hierin beschrieben und der konkreten Form des auf Stachelbeergewächsenen basierenden Bestandteils wie hierin beschrieben führt auch eine Submerskultur zu sehr angenehmen Aromen.

Als besonders vorteilhaft in Bezug auf die herstellbaren Aromastoffe hat sich ein Umsetzen von Pflanzenteilen der Johannisbeere enthaltendem Umsetzungsmedium wie hierin beschrieben mit Zusatz einer Aspartatquelle wie hierin beschrieben mithilfe eines Ständerpilzes wie hierin beschrieben erwiesen. Anstelle einer Aspartatquelle kann auch, wie vorstehend ausgeführt, eine pH-puffernde Komponente zu dem Umsetzungsmedium hinzugefügt werden, um den pH-Wert über die Dauer des Umsetzens in dem hier beschriebenen pH-Wertbereich zu halten. Insbesondere die Kultivierung von *W. cocos* auf Johannisbeertrester der Sorte Titania liefert einen blumigen und fruchtigen Geruch, der an Walderdbeere erinnert. Bei *W. cocos* handelt es sich um einen Speisepilz, der auch in der traditionellen chinesischen Medizin genutzt wird. Neben zahlreichen Minorkomponenten werden Linalool, Benzaldehyd und Methylanthranilat als Schlüsselaromakomponenten gebildet.

Das erfindungsgemäße Verfahren eignet sich insbesondere für die Herstellung eines Aromastoffes oder einer Mischung von Aromastoffen, die mindestens 1, bevorzugt 2 oder 3, weiter bevorzugt 4 oder 5 und am meisten bevorzugt 6, 7 oder 8 Verbindung enthält, die unter 2-Octanon, 2-Nonanon, Linalooxiden, Benzaldehyd, Geraniol, 2-Octanol, Methylanthranilat und Linalool ausgewählt ist. Besonders bevorzugt sind Verfahren, die sich mit der Herstellung von wenigstens 1 Verbindung, bevorzugt 2 und weiter bevorzugt alle Verbindungen von Linalool, Benzaldehyd und Methylanthranilat befassen. Diese Verbindungen sind besonders charakteristisch für die mit dem Umsetzungsmedium wie hierin beschrieben herstellbare Mischung von Aromastoffen. Insbesondere Methylanthranilat zeigt einen intensiv fruchtigen Geruch nach Walderdbeeren.

Die genaue Zusammensetzung in Art und Intensität der hergestellten Aromastoffe (hier auch als Aromakomposition bezeichnet) kann dabei dadurch beeinflusst werden, indem der Zeitpunkt des Gewinnens des Aromastoffs oder der Mischung von Aromastoffen variiert wird. In diesem Zusammenhang haben sich Erntezeitpunkte in erfindungsgemäßen Verfahren bewährt, in denen das Gewinnen des Aromastoffs oder der Mischung von Aromastoffen 3 bis 14 Tage, vorzugsweise 5 bis 14 Tage und insbesondere 9 bis 13 Tage nach Beginn des Inkontaktbringens erfolgt. Abgesehen davon ist auch vorgesehen, den Zeitpunkt des Gewinnens des Aromastoffs oder der Mischung von Aromastoffen in Abhängigkeit von einer vorbestimmten Aromakomposition festzulegen. Dadurch ist es unter Anderem möglich, zu einem Zeitpunkt zu ernten, an dem eine besonders angenehme Mischung an Aromastoffen vorliegt und/oder ein besonders angenehmer Aromastoff gegenüber möglicherweise weiteren gebildeten Aromastoffen deutlich überwiegt.

Des Weiteren betrifft die vorliegende Erfindung die Verwendung eines Umsetzungsmediums mit einem auf Pflanzenteilen der Stachelbeergewächse basierenden Bestandteils wie hierin beschrieben und/oder eines Biokatalysators wie hierin beschrieben zum Herstellen eines Aromastoffes oder einer Mischung von Aromastoffen wie hierin beschrieben. Insbesondere handelt es sich bei dem auf Pflanzenteilen der Stachelbeergewächse basierenden Bestandteil um einen Bestandteil, der auf Pflanzenteilen der schwarzen Johannisbeere basiert, wie Blätter oder Trester davon wie hierin beschrieben. Bei dem Biokatalysator handelt es sich insbesondere um einen Pilz aus der Abteilung der Ständerpilze wie hierin beschrieben.

Außerdem betrifft die vorliegende Erfindung eine Zusammensetzung, die insbesondere gemäß dem Verfahren wie hier beschrieben hergestellt (herstellbar) ist, umfassend 2 und insbesondere alle Verbindungen, die aus der Gruppe bestehend aus Linalool, Benzaldehyd und Methylanthranilat ausgewählt sind. Erfindungsgemäße Zusammensetzungen mit einem volumenbezogenen Gewichtsverhältnis von Linalool zu Benzaldehyd zwischen 100:1 und 4:1 und/oder einem volumenbezogenen Gewichtsverhältnis von Linalool zu Methylanthranilat zwischen 50:1 und 2:1 und/oder einem volumenbezogenen Gewichtsverhältnis von Benzaldehyd zu Methylanthranilat zwischen 3:1 und 1:10 sind bevorzugt. Erfindungsgemäße Zusammensetzungen enthalten ferner vorzugsweise mindestens eine weitere Verbindung, bevorzugt 2 oder 3, weiter bevorzugt 4 oder 5 und am meisten bevorzugt 6, 7 oder 8 Verbindungen, die aus der Gruppe bestehend aus 2-Octanon, 2-Nonanon, Linalooxiden, Benzaldehyd, Geraniol, 2-Octanol, Methylanthranilat und Linalool ausgewählt ist/sind.

Ferner betrifft die vorliegende Erfindung die Verwendung erfindungsgemäßer Zusammensetzungen wie hierin beschrieben als oder in einer der Ernährung, der Kosmetik, der Hygiene oder dem Genuss dienende(n) Zubereitung, insbesondere als Aromastoffmischung (bevorzugt für die hierin beschriebenen Zwecke). Dementsprechend betrifft die vorliegende Erfindung auch der Ernährung, der Kosmetik, der Hygiene oder dem Genuss dienende Zubereitungen umfassend oder bestehend aus einer erfindungsgemäßen Zusammensetzung wie hierin beschrieben.

Nachfolgend wird die Erfindung anhand des nachfolgenden Beispiels näher erläutert.

### BEISPIEL

### 1. Experimenteller Teil

### 1.1 Materialien und Chemikalien

Die verwendeten Nebenströme der Johannisbeerindustrie wurden von der Hochschule Geisenheim University, Deutschland, bereitgestellt. Die Quellen der verwendeten Pilze der Abteilung Basidiomycota sind Tabelle 1 zu entnehmen.

**Tabelle 1: Übersicht über die getesteten Pilze, deren Herkunft und Kultivierungsmedium; DSMZ: Leibniz Institut DSMZ-Deutsche Sammlung von Mikroorganismen und Zellkulturen, Braunschweig, Deutschland; CBS: CBS-KNAW culture collection, Westerdijk Fungal Biodiversity Institute, Utrecht, Niederlande; S: Sammlung Institut für Lebensmittelchemie und Lebensmittelbiotechnologie der Justus-Liebig-Universität Gießen; MEA: Malzextraktagar; SNL: Standardnährlösungsagar.**

| Name | Stammnr. | Herkunft | Medium |
|---|---|---|---|
| *Agaricus campestris* | FP44 | S | SNL |
| *Agrocybe aegerita* | AAE3 | S | MEA |
| *Armillaria melea* | 2941 | DSMZ | MEA |
| *Bjerkandera adusta* | 4708 | DSMZ | MEA |
| *Coprinus comatus* | FP91 | S | MEA |
| *Cyathus limbatus* | 335.81 | CBS | MEA |
| *Flammulina velutipes* | 1658 | DSMZ | MEA |
| *Gloeophyllum odoratum* | 12011 | DSMZ | MEA |
| *Hypholoma fasciculare* | 2902 | DSMZ | MEA |
| *Irpex consors* | 7382 | DSMZ | MEA |
| *Laetiporus sulphureus* | 1014 | DSMZ | MEA |
| *Lentinula edodes* | 389.89 | CBS | MEA |
| *Lepista nuda* | 3347 | DSMZ | MEA |
| *Lycoperdon pyriforme* | 8676 | DSMZ | MEA |
| *Marasmius cohortalis* | 8257 | DSMZ | MEA |
| *Mycena pseudocorticola* | 108 | S | MEA |
| *Mycetinis scorodonius* | 137.83 | CBS | MEA |
| *Panellus serotinus* | 5204 | DSMZ | MEA |
| *Phanerochaete chrysosporium* | 481.73 | CBS | MEA |
| *Pleurotus flabellatus* | 8299 | DSMZ | MEA |
| *Pleurotus sapidus* | 8266 | DSMZ | MEA |
| *Sparassis crispa* | FP32 | S | MEA |
| *Stereum hirsutum* | Bda11 | S | MEA |
| *Trametes suaveolens* | 5237 | DSMZ | MEA |
| *Tyromyces chioneus* | 5242 | DSMZ | MEA |
| *Wolfiporia cocos* | 279.55 | CBS | MEA |

### 1.2 Stammhaltung

Die zu untersuchenden Pilze (Tabelle 1) wurden auf Malzextraktagarplatten (MEA) bzw. Standardnährlösungsagarplatten (SNL) zur Stammhaltung kultiviert. Alle Medien wurden für 20 min bei 121 °C dampfsterilisiert. Die Medien setzten sich wie folgt zusammen:
MEA: 20 g·L⁻¹ Malzextrakt (ME), 15 g·L⁻¹ Agar-Agar in entmin. Wasser.
SNL: 30 g·L⁻¹ Glucose-Monohydrat, 15 g·L⁻¹ Agar-Agar, 4,5 g·L⁻¹ Asparagin-Monohydrat, 3 g·L⁻¹ Hefeextrakt, 1,5 g·L⁻⁻¹ Kaliumdihydrogenphosphat, 0,5 g·L⁻¹ Magnesiumsulfat-Hydrat, 400 µg·L⁻¹ EDTA, 90 µg·L⁻¹ Zink(II)-sulfat-Heptahydrat, 80 µg·L⁻¹ Eisen(III)-chlorid-Hexahydrat, 30 µg·L⁻¹ Mangan(II)-sulfat-Monohydrat, 5 µg·L⁻¹ Kupfer(II)-sulfat-Pentahydrat, in entmin. Wasser und auf pH 6,0 eingestellt.

Zur Kultivierung wurde ein ca. 0,5 cm² großes Myzelstück einer zu 80% bewachsenen Agarplatte auf eine neue Agarplatte gesetzt, die Platte mit Parafilm® verschlossen und bei 24 °C im Dunkeln inkubiert.

### 1.3 Screening auf Reststoffströmen der Schwarzen Johannisbeere

Die Kultivierung der Pilze der Abteilung Basidiomycota auf den Reststoffströmen Blätter und Trester der Schwarzen Johannisbeere erfolgte in Submers-, Emers- und Festbettkultur. Zunächst wurden die Reststoffströme als alleinige Nährstoffquelle für die 26 getesteten Pilze eingesetzt.

Für die Kultivierung der Pilze in Emerskultur wurden Agar-Platten mit Substrat in verschiedenen Konzentrationen hergestellt. Je nach Substrat wurden unterschiedliche Konzentrationen an Agar benötigt. Diese wurden mit den 26 ausgewählten Pilzen (Tabelle 1) analog zur Stammhaltung angeimpft. Nach 4 bis 7 d, je nach Wachstum der Pilze, wurden die Platten erstmalig auf ihre sensorischen Eigenschaften hin untersucht. Zur Emerskultivierung von *W*. *cocos* auf Johannisbeertrester für die weiterführenden Experimente wurden 3% Trester in 30% des entmin. Wassers und 3% Agar in 70% des entmin. Wassers autoklaviert und vor dem Gießen gemischt. Jede Platte enthielt circa 16 mL Medium.

Für die Kultivierung der Pilze in Submerskultur wurden zunächst Vorkulturen (100 mL) in 2%-igem ME-Medium angesetzt. Dies erfolgte, indem ein ca. 1 cm² großes, mit Myzel überwachsenes Agarstück von der Stammhaltungsplatte in einen Erlenmeyerkolben mit ME-Medium überführt und dort mittels Dispergiergerät (Ultraturrax, 10.000 rpm, 30 s) homogenisiert wurde. Die Vorkultur wurde für 8 d bei 24 °C unter Lichtausschluss bei 150 rpm inkubiert. Zur Inokulation der Hauptkultur wurde die Vorkultur homogenisiert (Ultraturrax, 10.000 rpm, 30 s), zentrifugiert (3.000 g, 10 min) und der Überstand dekantiert. Das Myzel wurde zweimal mit sterilem Wasser gewaschen und anschließend in 100 mL sterilem Wasser suspendiert. Von dieser Suspension wurden 4 mL zu 40 mL Hauptkulturmedium gegeben. Das Hauptkulturmedium bestand aus 15 g·L⁻¹ bzw. 30 g·L⁻¹ Substrat in entmineralisiertem Wasser. Diese Kulturen wurden bei 24 °C im Dunkeln bei 150 rpm kultiviert. Die sensorische Analyse erfolgte ab dem 3. Kulturtag durch Riechen direkt am Kolben. Zum Vergleich wurde eine Hauptkultur in ME-Medium sowie nicht-inokulierte Hauptkulturmedien als Blindwerte mitgeführt.

### 1.4 Optimierung der Kultivierung auf Reststoffströmen der Schwarzen Johannisbeere

Zur Optimierung des Aromaprofils und der Intensität wurden weitere Nährstoffquellen zu dem Substrat gegeben. Neben der Einstellung des pH-Werts wurden Glucose, Spurenelemente (Mg²⁺, Zn²⁺, Fe3+, Mn²⁺, Cu²⁺, K⁺, Cl⁻, SO₄²⁻, PO₄³⁻), Mononatrium-L-Aspartat (Asp) und andere Aminosäuren supplementiert. Außerdem wurden die Substratkonzentrationen der Johannisbeer-Nebenströme variiert.

### 1.5 Sensorische Analyse

Die sensorischen Analysen wurden von einem trainierten Panel (n = 4) durchgeführt, um eine Vorauswahl der Pilz-Substrat-Kombinationen vorzunehmen. Dazu wurden die Kulturen in einer "einfach beschreibenden Prüfung" mit Bewertung des Gesamteindruckes an mehreren Kulturtagen untersucht. Die Skala für die Bewertung des Gesamteindruckes umfasste sechs Stufen von sehr negativ (0) zu sehr positiv (5). Die Identifizierung der Aromen erfolgte mittels GC-MS-O.

### 1.6 Bestimmung des Myzelwachstums

Während der Kultivierung wurde das Wachstum der Pilze täglich durch Einzeichnen der bewachsenen Fläche auf der Rückseite der Agarplatte dokumentiert und der Durchmesser bestimmt. Daraus wurde die bewachsene Myzelfläche berechnet.

### 1.7 pH-Wert-Bestimmung

Um den pH-Wert-Verlauf der Submerskulturen zu bestimmen, wurde ein Aliquot (2 mL) entnommen und der pH-Wert mit Hilfe eines pH-Meters bestimmt. Bei den Emerskulturen wurde der pH-Wert der Kulturen bestimmt, indem der Agar einer Platte nach Zusatz von 15 mL entmin. Wasser mittels Ultraturrax (10.000 rpm, 10 s) homogeniert, anschließend zentrifugiert (10 min, 4.000 g) und der pH-Wert im Überstand mittels eines pH-Meters gemessen wurde.

### 1.8 GC-MS-O Analyse

Die Aromen der Kulturen wurden mittels *stir bar sorptive extraction* (SBSE) für 60 min bei 24 °C extrahiert. Dazu wurden 10 mm Magnetrührfische mit 0,5 mm PDMS-(Polydimethylsiloxan)-Beschichtung (Twister, GERSTEL, Mühlheim an der Ruhr, Deutschland) mit einem Magneten im Kopfraum des Kultivierungsgefäßes fixiert. Nach der Inkubation wurden die Magnetrührfische mit einem Magnetstab entfernt, mit entmin. Wasser gespült, mit einem fusselfreien Tuch getrocknet und in einen konditionierten Thermal Desorption Unit (TDU) Liner (GERSTEL) eingesetzt. Die TDU-Desorption erfolgte splitlos. Die Analyten wurden mit einem Glasverdampferrohr mit silanisierter Glaswatte (GERSTEL) in einem Cold Injection System 4 (CIS) (GERSTEL) cryofokussiert. Die gaschromatographische Analyse erfolgte mittels GC-MS-O, bestehend aus einem 7890B GC (Agilent Technologies, Waldbronn, Deutschland) gekoppelt mit einem 5977B MSD (Agilent Technologies) sowie einem Olfaktometrie-Detektorport (ODP) (GERSTEL). Als polare Säule wurde eine Agilent J&W VF-WAXms (30 m × 0,25 mm ID × 0,25 µm), als unpolare Trennphase eine Agilent J&W DB-5ms (30 m × 0,25 mm ID × 0,25 µm) genutzt. Als Trägergas wurde Helium mit einer konstanten Flussrate von 1,56 mL·min⁻¹ verwendet. Der Gasfluss wurde mit einem Split von 1:1 in das MS und in den ODP geleitet. Weitere Einstellungen sind im Folgenden aufgeführt: Septum Purge-Fluss 3 mL·min⁻¹, Scan-Modus total ion current (TIC), Scan-Bereich m/z 33 - 300, lonisationsenergie 70 eV, El-Quellen-Temperatur 230 °C, Quadrupol-Temperatur 150 °C, MS Transferline-Temperatur 250 °C, He Quench-Gas 2,25 mL·min⁻¹, N₂ Kollisionsgas 1,5 mL·min⁻¹, ODP 3 Transferline-Temperatur 250 °C, ODP Mischkammer-Temperatur 150 °C, ODP Makeup-Gas N₂.

Zur Untersuchung der Veränderungen der relativen Intensitäten der Aromen und zur Identifizierung mittels VF-WAXms wurde die Ofentemperatur nach 3 min bei 40 °C mit 10 °C·min⁻¹ auf 150 °C, dann mit 20 °C·min⁻¹ auf 240 °C erhöht und die Temperatur für 7 min gehalten. Das TDU-Temperaturprogramm heizte von 40 °C (0,5 min) mit 360 °C·min⁻¹ auf 250 °C (10 min). Am CIS wurde nach einer Haltezeit von 0,5 min mit 12 °C·s⁻¹ von -20 °C auf 250 °C erhitzt und die Temperatur für 5 min gehalten. Das CIS-Splitverhältnis wurde zwischen splitlos und 50:1 variiert.

Zur semiquantitativen Abschätzung und zur Bestimmung der Retentionsindizes mittels DB-5ms wurde der Ofen nach 3 min bei 40 °C mit 5 °C·min⁻¹ auf 320 °C erhitzt und die Temperatur für 7 min gehalten. Die TDU wurde abweichend mit 120 °C·min⁻¹ auf 250 °C aufgeheizt. Die Cryofokussierung mittels CAS erfolgte abweichend bei -100 °C. Das Splitverhältnis am CIS wurde zwischen splitless und 50:1 variiert.

### 1.9 Identifizierung von Aromastoffen

Zur Identifizierung der Subtanzen wurden je 10-30 mg der Referenzsubstanz abhängig von der Löslichkeit mit 50-1000 µL DMSO (Carl Roth, Karlsruhe Deutschland) versetzt und ggf. ad 1 mL mit entmin. Wasser aufgefüllt. Zur Analyse wurden die folgenden Aromastoffe verwendet: 2-Nonanon ≥ 99% (Acros Organics, Geel, Belgien), Linalool 97% (Acros Organics), Methylanthranilat 99% (Acros Organics), (+)-2-Octanol 98% (Alfa Aesar, Haverhill, USA), Geraniol 97% (Alfa Aesar), 2-Octanon 98% (Sigma Aldrich, St. Louis, USA), Linalooloxid Isomerengemisch (Sigma Aldrich), 2-Undecanon pur (Honeywell Fluka, Bukarest, Rumänien), Benzaldehyd pur (AppliChem, Darmstadt, Deutschland). Daraus wurden 1:200 und 1:1000 Verdünnungen mit Wasser hergestellt und 0,5 mL der Lösungen auf Agar (1,5% Agar in entmin. Wasser) ausplattiert. Nach einer Stunde wurde ein Twister® mit einem Magneten im Kopfraum befestigt und mit den im vorhergehenden Punkt 1.8 beschriebenen Methoden mittels GC-MS analysiert. Die Retentionsindizes wurden nach van den Dool und Kratz berechnet (van Den Dool, H.; Dec. Kratz, P., Journal of Chromatography A, (1963) 11, 463).

### 1.10 Semiquantitative Abschätzung der Konzentrationen von Methylanthranilat, Linalool und Benzaldehyd

Zur semiquantitativen Abschätzung wurden Linalool, Methylanthranilat und Benzaldehyd in entmin. Wasser gelöst. Für Benzaldehyd wurden Standards mit Konzentrationen von 3, 4, 8, 12 bzw. 16 mg·L⁻¹, für Methylanthranilat mit 9, 14, 19, 41, 80 mg·L⁻¹ und für Linalool mit 120, 241, 361 und 722 mg·L⁻¹ hergestellt. Davon wurden jeweils 0,5 mL auf einer Agarplatte (Ø 10 cm, 16 mL 1,5% Agar in entmin. Wasser) ausplattiert. Nach einer Stunde wurde mit Hilfe eines Magneten ein Twister® im Kopfraum befestigt und analog zu den Proben analysiert. Durch Bestimmung der Peakflächen, die mittels Extracted Ion Chromatogram (EIC) eines für die Substanz spezifischen Fragments berechnet wurden, wurden die in der Probe detektierten Intensitäten mit den Intensitäten der Standardsubstanzen verglichen und die Proben so einem Konzentrationsbereich zugeordnet.

### 2. Ergebnisse

Zur Untersuchung der Biotransformation von Nebenströmen der Schwarzen Johannisbeere zur Synthese natürlicher Aromen wurden insgesamt 26 Pilze der Abteilung Basidiomycota getestet. Tabelle 2 gibt eine Übersicht über die Bewertung der Geruchseindrücke der getesteten Pilz-Substrat-Kombinationen in Emers- und Submerskultur.

**Tabelle 2: Sensorische Bewertung des Gesamteindruckes der Pilz-Substrat-Kombinationen zwischen 0 (sehr negativ) und 5 (sehr positiv). -: nicht durchgeführte Kultivierungen.**

| Name | Emerskultur Blätter | Emerskultur Trester | Submerskultur Blätter | Submerskultur Trester |
|---|---|---|---|---|
| *A. campestris* | 2 | 1 | 4 | 1 |
| *A. aegerita* | 0 | 1 | 5 | 4 |
| *A. melea* | 0 | - | - | - |
| *B. adusta* | 2 | 2 | 2 | 2 |
| *C. comatus* | 0 | 1 | 5 | 5 |
| *C*. *limbatus* | 0 | - | - | - |
| *F. velutipes* | - | - | 1 | 1 |
| *G. odoratum* | 5 | 5 | 5 | 5 |
| *H. fasciculare* | 3 | - | 4 | 4 |
| *I. consors* | 3 | 2 | 2 | 2 |
| *L. sulphureus* | 5 | 0 | 4 | 1 |
| *L. edodes* | 3 | 0 | 0 | 0 |
| *L. nuda* | - | - | 1 | 0 |
| *L. pyriforme* | 1 | 1 | 0 | 4 |
| *M. cohortalis* | - | - | 0 | 0 |
| *M. pseudocorticola* | 5 | 5 | 5 | 0 |
| *M. scorodonius* | 5 | 0 | 5 | 5 |
| *P. serotinus* | - | - | 3 | 0 |
| *P. chrysosporium* | 0 | 0 | 3 | 1 |
| *P. flabellatus* | 0 | 1 | 5 | 5 |
| *P. sapidus* | 5 | 1 | 5 | 2 |
| *S*. *cr*/*spa* | 3 | 0 | 5 | 0 |
| *S. hirsutum* | 0 | 1 | 1 | 0 |
| *T. suaveolens* | 4 | 0 | 0 | 3 |
| *T. chioneus* | - | - | 3 | 1 |
| *W*. *cocos* | 5 | 5 | 5 | 5 |

Anhand der sensorischen Analyse wurden mehrere interessante Pilz-Substrat-Kombinationen identifiziert. Die Pilze *G. odoratum* und *W. cocos* zeigten auf allen getesteten Substraten in allen getesteten Kultivierungsarten sehr intensive Geruchseindrücke. *G. odoratum* produzierte hierbei ein citrusartiges Aroma, das Aroma von *W. cocos* war blumig, fruchtig und erinnerte an Walderdbeeren.

Zur genaueren Analyse des Wachstumsverhaltens von *W. cocos* wurde dieser auf unterschiedlichen Medien kultiviert. Neben dem Trester wurde Malzextraktagar gewählt, welcher als Vollmedium gut für das Wachstum des Pilzes geeignet war. Ein Zusatz von Mononatrium-L-Aspartat führte bei der Kultivierung auf Trester zu einem besonders intensiven Aroma. Neben den Gerüchen unterschieden sich die Kulturen sowohl optisch als auch durch ihr Wachstum (vgl. Figur 1).

Bei Zugabe von Aspartat färbten sich sowohl das Myzel als auch das Nährmedium braun. Außerdem war das Wachstum im Vergleich zu einem Referenzmedium reduziert (vgl. Figur 2). Dabei wurde weniger Biomasse und ein sehr flaches Myzel gebildet.

Bei Zugabe von 0,624% Mononatrium-L-Aspartat fiel der pH-Wert weniger stark ab als bei den Kulturen ohne Aspartat (vgl. Figur 3). Sowohl bei der Fermentation auf Trester als auch auf MEA-Medium sank der pH-Wert über den Kulturverlauf hinweg auf etwa 2,5. Im Gegensatz dazu war beim Medium aus Trester mit Aspartatzusatz kein Abfall des pH-Wertes zu beobachten. Bei diesen Kulturen war der Geruch nach Walderdbeeren besonders intensiv.

Zur Analyse der Aromen-Zusammensetzung wurden SBSE-Analysen in Kombination mit GC-MS-O durchgeführt. Als besonders interessant wurden dabei die Fermentationen von *W*. *cocos* auf Johannisbeertrester mit Zusatz von 0,624% Mononatrium-L-Aspartat identifiziert. Die detektierten Aromen unterschieden sich signifikant von denen, die bei der Fermentation auf MEA oder Johannisbeertrester bzw. Johannisbeerblättern ohne Aspartatzusatz nachgewiesen wurden.

Im MEA-Medium wurden insbesondere C8-Aromen wie 3-Octanon, 1-Octen-3-ol, 1-Octanol und (*E*)-2-Octen-1-ol detektiert (vgl. Figur 4). Diese sind als typische Pilzaromen beschrieben (Hofrichter, M., ed., Industrial Applications, (2011), Berlin, Heidelberg, Springer Berlin Heidelberg). Dies korrelierte gut mit dem olfaktorischen Eindruck, der als pilzig, säuerlich und nach tropischen Früchten riechend beschrieben wurde.

Im Vergleich dazu unterschied sich das Aroma bei Kultivierung von *W*. *cocos* auf Johannisbeertrester mit Aspartat deutlich. Die Kultur begann bereits nach wenigen Tagen blumig und frisch zu riechen. Des Weiteren waren keine Aromen, die an Pilz erinnerten, wahrnehmbar. Nach ca. 10 Kulturtagen intensivierte sich der Geruch mit blumigen Noten und einem intensiven fruchtigen Aroma, das an Walderdbeeren erinnerte.

Die Signalintensitäten der Aromen waren bei Kultivierung auf dem mit Aspartat-supplementierten Johannisbeertrester deutlich höher als bei der Kultivierung auf MEA (vgl. Figur 5). Besonders hoch waren die Intensitäten von Linalool, was gut mit dem blumigen Geruch der Kultur korrelierte.

Neben Linalool wurden mittels GC-MS-O-Untersuchungen weitere Verbindungen als charakteristisch für das Aroma der Pilz-Substrat-Kombination identifiziert. Insbesondere Methylanthranilat zeigte einen intensiv fruchtigen Geruch nach Walderdbeeren.

Die Komplexität der Aromenzusammensetzung wurde außerdem durch die olfaktorisch identifizierten Verbindungen unterstrichen. Dazu wurden Emerskulturen von *W*. *cocos* mittels GC-MS-O an definierten Kulturtagen untersucht, um besonders potente Aromen nachzuweisen (vgl. Figur 6).

Bei der so erfolgten olfaktorischen Bewertung der Kultur waren die Substanzen Linalool und Methylanthranilat sehr stark wahrnehmbar. Auch Benzaldehyd wurde mit seinem charakteristischen Geruch nach Bittermandeln mittels ODP wahrgenommen. Neben diesen Schlüsselaromastoffen war eine Vielzahl weiterer Gerüche wahrnehmbar, für die eine Substanzzuordnung noch aussteht.

In Tabelle 3 sind Vorschläge für ausgewählte, in der Probe enthaltene Substanzen mit deren Retentionsindizes (RI) nach van den Dool und Kratz und dem Geruch im Vergleich mit den Retentionsindizes der Standardsubstanzen und den in der Literatur beschriebenen Gerüchen aufgeführt. Die zugehörigen Massenspektren (nicht dargestellt) bestätigten durch Vergleich mit entsprechenden Vergleichsspektren aus der NIST-Datenbank die Bildung von 2-Octanon, 2-Nonanon, Linalooloxiden, Benzaldehyd, Geraniol, 2-Octanol, Methylanthranilat und Linalool anhand Proben aus einer Emerskultur von *W. cocos* auf 3% Trester + 0,624% Mononatrium-L-Aspartat.

**Tabelle 3: Retentionsindizes (RI) nach van den Dool und Kratz und Gerüche von den in der Probe detektierten Substanzen im Vergleich zu Standardsubstanzen (STD) und in der Literatur beschriebenen Gerüchen. VF-WAXms und DB-5ms bezeichnen die jeweils verwendete Trennphase (vgl. Experimenteller Teil, Punkt 1.8).**

| | VF-WAXms | | DB-5ms | | Geruch | |
|---|---|---|---|---|---|---|
| | Probe | STD | Probe | STD | GC-MS-O | Literatur |
| 2-Nonanon | 1393 | 1393 | 1093 | 1093 | grün | frisch, süßlich, grün, grasig, erdig ^{[6]} |
| 2-Octanol | 1417 | 1417 | 1005 | 1004 | grün | frisch, würzig, grün, holzig, krautig ^{[7]} |
| 2-Octanon | 1290 | 1290 | 994 | 993 | grün | erdig, krautig, holzig ^{[8]} |
| Benzaldehyd | 1536 | 1536 | 968 | 967 | Marzipan, süßlich | süßlich, Mandel, Kirsche ^{[9]} |
| Geraniol | 1848 | 1848 | 1252 | 1252 | grün | süßlich, blumig, Geranie, fruchtig ^{[10]} |
| Linalool | 1547 | 1547 | 1103 | 1102 | blumig, frisch, Zitrus | Zitrus, floral, süßlich, nach Rose, grün, Blaubeere ^{[11]} |
| Linalooloxid I | 1446 | 1445 | 1075 | 1074 | grün, Tanne, dumpf | floral, krautig, erdig, grün ^{[12]} |
| Linalooloxid II | 1474 | 1473 | 1090 | 1090 | dumpf | |
| Methylanthranilat | 2271 | 2269 | 1345 | 1345 | fruchtig, Walderdbeere, süßlich | fruchtig, Traube, Orangenblüte ^{[13]} |

Sowohl die Retentionsindizes als auch die Massenspektren und Gerüche von Probe und Standard stimmten jeweils gut überein.

Mittels Kopfraum-SBSE wurde die Bildungskinetik für die Hauptaromen im Abstand von 24 h über einen Zeitraum von 14 d erfasst (vgl. Figur 7).

Einige aromawirksame Stoffe, wie Benzaldehyd, Linalool, Linalooloxid I, II und 2-Undecanon wurden nahezu ausschließlich auf dem Medium aus Trester und Aspartat (3TT+Asp) gebildet. Andere Verbindungen, wie 2-Nonanon, Geraniol und Methylanthranilat wurden sowohl auf dem Medium aus Trester und Aspartat als auch auf dem Medium aus MEA und Aspartat (MEA+Asp) gebildet.

Die Daten der Aromaanalytik (vgl. Figuren 8 und 9) korrelierten gut mit denen der sensorischen Analyse, in der der charakteristische Geruch am 10. Kulturtag als am intensivsten beschrieben wurde.

Als besonders charakteristisch für das Aroma wurden Linalool, Methylanthranilat und Benzaldehyd wahrgenommen. Aus diesem Grund wurde eine semiquantitative Abschätzung der Aromakonzentrationen bei Kultivierung von *W. cocos* auf Trester mit Aspartat nach 10 d vorgenommen (vgl. Figur 10).

Anhand der Kalibrierreihen ließen sich für Linalool Werte von 95-135 µg pro Platte, für Benzaldehyd ca. 5 µg und für Methylanthranilat 5-20 µg pro Platte abschätzen. Unter Berücksichtigung des Plattenvolumens von ca. 16 mL entsprach dies ca. 7.000 ± 1.500 µg·L⁻¹ Linalool, 350 ± 50 µg·L⁻¹ Benzaldehyd und 700 ± 350 µg·L⁻¹ Methylanthranilat.

## Patentansprüche

1. Verfahren zum Herstellen eines Aromastoffes oder einer Mischung von Aromastoffen, umfassend die Schritte:
Bereitstellen eines Umsetzungsmediums mit einem auf Pflanzenteilen der Stachelbeergewächse basierenden Bestandteil;
Inkontaktbringen des Umsetzungsmediums mit einem Biokatalysator;
Umsetzen des zumindest auf Pflanzenteilen der Stachelbeergewächse basierenden Bestandteils zu dem Aromastoff oder der Mischung von Aromastoffen mithilfe des Biokatalysators; und gegebenenfalls
Gewinnen des Aromastoffs oder der Mischung von Aromastoffen.

2. Verfahren nach Anspruch 1, wobei das Stachelbeergewächs aus der Gruppe der Arten der Johannisbeeren, Stachelbeeren und deren Kreuzungen ausgewählt ist,
wobei das Stachelbeergewächs bevorzugt aus der Gruppe der Arten der weißen oder roten Stachelbeere, der roten, weißen, schwarzen oder tiefschwarzen Johannisbeeren sowie deren Kreuzungen und Sorten ausgewählt ist.

3. Verfahren nach einem der vorangehenden Ansprüche, wobei der auf Pflanzenteilen der Stachelbeergewächse basierende Bestandteil aus der Gruppe bestehend aus Blättern, Knospen, Blatt- Knospen, Blüten-Knospen und Beerenfrüchte, bevorzugt Blättern und Beerenfrüchten und weiter bevorzugt reifen Beerenfrüchten, sowie jeweils deren Unterbestandteile, Extrakte und Trester, ausgewählt ist.

4. Verfahren nach einem der vorangehenden Ansprüche, wobei es sich bei dem Biokatalysator um wenigstens einen Pilz aus der Abteilung der Ständerpilze, oder um wenigstens eine Pilzzelle davon oder wenigstens eine katalytisch wirksame Einheit davon, handelt.

5. Verfahren nach Anspruch 4, wobei der wenigstens eine Pilz aus der Abteilung der Ständerpilze aus der Gruppe bestehend aus *A. campestris, A. aegerita, A. melea, B. adusta, C. comatus, C. limbatus, F. velutipes, G. odoratum, H. fasciculare, I. consors, L. sulphureus, L. edodes, L. nuda, L. pyriforme, M. cohortalis, M. pseudocorticola, M. scorodonius, P. serotinus, P. chrysosporium, P. flabellatus, P. sapidus, S. crispa, S. hirsutum, T. suaveolens, T. chioneus und W*. *cocos* ausgewählt ist, wobei der Pilz bevorzugt *W*. *cocos* oder *G. odoratum* ist.

6. Verfahren nach einem der vorangehenden Ansprüche, wobei der Aromastoff oder die Mischung von Aromastoffen mindestens eine Verbindung enthält, die aus der Gruppe bestehend aus 2-Octanon, 2-Nonanon, Linalooxiden, Benzaldehyd, Geraniol, 2-Octanol, Methylanthranilat und Linalool ausgewählt ist.

7. Verfahren nach einem der vorangehenden Ansprüche, wobei die Mischung von Aromastoffen wenigstens 2 und insbesondere alle 3 Verbindungen enthält, die aus der Gruppe bestehend aus Linalool, Benzaldehyd und Methylanthranilat ausgewählt sind.

8. Verfahren nach einem der vorangehenden Ansprüche, wobei das bereitgestellte Umsetzungsmedium eine Aspartatquelle umfasst und der Gehalt der Aspartatquelle vorzugsweise 0,1 % bis 5,0 %, bevorzugt 0,2 % bis 2,0 %, weiter bevorzugt 0,3 % bis 1,0 % und insbesondere 0,5 % bis 0,7 %, jeweils bezogen auf das Gesamtgewicht des Umsetzungsmediums beträgt.

9. Verfahren nach einem der vorangehenden Ansprüche, wobei der pH-Wert des Umsetzungsmediums während dem Umsetzen innerhalb eines Bereichs von pH 2 bis pH 7, vorzugsweise pH 4 bis pH 6 und insbesondere pH 4,2 bis pH 5,5 liegt.

10. Verfahren nach einem der vorangehenden Ansprüche, wobei das Umsetzen in Emerskultur oder Submerskultur, vorzugsweise Emerskultur, erfolgt.

11. Verwendung eines Umsetzungsmediums mit einem auf Pflanzenteilen der Stachelbeergewächse basierenden Bestandteil und/oder eines Biokatalysators zum Herstellen eines Aromastoffes oder einer Mischung von Aromastoffen.

12. Zusammensetzung, die vorzugsweise gemäß dem Verfahren nach einem der Ansprüche 1 bis 10 hergestellt ist, umfassend 2 und insbesondere alle 3 Verbindungen, die aus der Gruppe bestehend aus Linalool, Benzaldehyd und Methylanthranilat ausgewählt sind, vorzugsweise
mit einem volumenbezogenen Gewichtsverhältnis von Linalool zu Benzaldehyd zwischen 100:1 und 4:1 und/oder
einem volumenbezogenen Gewichtsverhältnis von Linalool zu Methylanthranilat zwischen 50:1 und 2:1 und/oder
einem volumenbezogenen Gewichtsverhältnis von Benzaldehyd zu Methylanthranilat zwischen 3:1 und 1:10.

13. Zusammensetzung nach Anspruch 12, ferner umfassend mindestens eine weitere Verbindung, die aus der Gruppe bestehend aus 2-Octanon, 2-Nonanon, Linalooxiden, Benzaldehyd, Geraniol, 2-Octanol, Methylanthranilat und Linalool ausgewählt ist.

14. Der Ernährung, der Kosmetik, der Hygiene oder dem Genuss dienende Zubereitung umfassend oder bestehend aus einer Zusammensetzung gemäß einem der Ansprüche 12 oder 13.

15. Verwendung einer Zusammensetzung gemäß einem der Ansprüche 12 oder 13 als oder in einer der Ernährung, der Kosmetik, der Hygiene oder dem Genuss dienende(n) Zubereitung, vorzugsweise als Aromastoffmischung.
